# EUROPÄISCHE PATENTSCHRIFT

(11) **EP 1 140 221 B1**
(45) Veröffentlichungstag und Bekanntmachung des Hinweises auf die Patenterteilung: **28.08.2002**
(21) Anmeldenummer: 98966644.1
(22) Anmeldetag: 18.12.1998
(51) Int. Cl.: A61L 2/26, A61L 2/00

(54) **STERILISIERBEHÄLTER**
STERILIZATION CONTAINER
RECIPIENT DE STERILISATION

(43) Veröffentlichungstag der Anmeldung: 10.10.2001
(73) Patentinhaber: Aesculap AG & Co. KG, 78532 Tuttlingen (DE)
(72) Erfinder: GABELE, Lorenz, D-88605 Sauldorf (DE)
(74) Vertreter: Böhme, Ulrich, Dr. Dipl.-Phys.
(86) Internationale Anmeldenummer: EP9808331
(87) Internationale Veröffentlichungsnummer: WO00037116

(56) Entgegenhaltungen:
- EP-A- 0 483 104
- DE-A- 3 710 156
- US-A- 4 900 519
- US-A- 4 959 199
- US-A- 5 384 103
- US-A- 5 441 707
- US-A- 5 792 422

## Beschreibung

Die Erfindung betrifft einen Sterilisierbehälter mit einem wannenförmigen Unterteil und mit einem dichtend auf dieses aufsetzbaren Deckel.

Sterilisierbehälter dieser Art werden verwendet, um chirurgische Instrumente aufzunehmen, die in diesen Sterilisierbehältern sterilisiert und davor und danach aufbewahrt werden. Es ist üblich, diese Sterilisierbehälter aus Metall herzustellen, beispielsweise aus Aluminium. Die Oberfläche derartiger Sterilisierbehälter wird aus Gründen des Oberflächenschutzes und aus Gründen der Kennzeichnungsmöglichkeit häufig mit einer Beschichtung versehen, und zwar vorzugsweise mit einer Eloxaloberfläche, die gegebenenfalls auch farbig ausgebildet werden kann.

Allerdings ergeben sich bei einer derartigen Oberflächenausgestaltung Probleme beim Sterilisieren, da durch die aggressiven Sterilisationsbedingungen diese Oberfläche in ihrer Struktur und auch in ihrer Farbgestaltung verändert wird. Schwierigkeiten ergeben sich auch bei der Reinigung der Sterilisierbehälter, da derartige Eloxaloberflächen nur mit bestimmten Reinigungsmitteln behandelt werden dürfen, die grundlegend verschieden sind von denen, die zur Reinigung der chirurgischen Instrumente selbst eingesetzt werden.

In der US-A-4 900 519 ist ein Sterilisierbehälter mit dichtendem Deckel beschrieben, der aus vorzugsweise durchsichtigem Kunststoff besteht. Dieser enthält gut wärmeleitende Teilchen oder ist mit einer gut wärmeleitenden Beschichtung versehen, um die Wärmeleitfähigkeit des Verbundmaterials zu erhöhen und so eine Kondensat-Akkumulation zu verhindern.

Die US-A-5 792 422 offenbart Sterilisiereinschübe aus Metall oder Kunststoffen, bzw. Kunststoffe mit wärmeleitenden Füllstoffen, wobei die Resistenz gegen Wasserstoffperoxid und die Wärmeleitfähigkeit für die Materialwahl entscheidend ist. Die Einschübe kommen in Behältern mit dichtenden Deckeln aus nicht näher angegebenem Material zum Einsatz.

In der US-A-5 384 103 ist ein Sterilisiereinschub mit Deckel aus hitzebeständigem Material, insbesondere aus eloxiertem Aluminium, beschrieben. Die Einschübe kommen in geschlossenen Behältern aus nicht näher angegebenem Material zum Einsatz.

Auch bei diesen bekannten Behältern werden die beschriebenen Probleme beim Sterilisieren nicht mit Sicherheit vermieden.

Es ist daher Aufgabe der Erfindung, einen gattungsgemäßen Sterilisierbehälter so auszugestalten, daß seine Oberfläche gegen chemische und gegen Temperatureinflüsse, gegen Abrieb, gegen Kratzer und/oder gegen Lärmentwicklung geschützt ist, und zwar unabhängig vom Substratmaterial.

Diese Aufgabe wird bei einem Sterilisierbehälter der eingangs beschriebenen Art erfindungsgemäß dadurch gelöst, daß das Unterteil und/oder der Deckel allseitig oder außenseitig mit Polytetrafluorethylen beschichtet sind.

Eine derartige Beschichtung hat sich als überraschend widerstandsfähig herausgestellt, so daß in dieser Weise beschichtete Sterilisierbehälter eine große Anzahl von Sterilisationszyklen praktisch unbeschadet überstehen können.

Zusätzlich können Beschlagteile des Sterilisierbehälters und/oder Hinweisschilder ebenfalls mit Polytetrafluorethylen beschichtet sein.

Besonders vorteilhaft ist es, wenn dem Polytetrafluorethylen-Beschichtungsmaterial Farbstoffe beigemischt sind, dadurch wird es möglich, den gesamten Behälter oder Behälterteile farbig auszugestalten. Diese farbige Beschichtung übersteht Sterilisiervorgänge ebenfalls ohne jede Veränderung, und selbst nach 200 Sterilisationszyklen sieht ein solcher Behälter noch aus wie neu.

Die Beschichtung hat weiterhin den Vorteil, daß sie nichthaftende Eigenschaften hat, so daß Verschmutzungen sich nicht an Behälterteilen festsetzen. Dies ist gerade bei Sterilisationsbehältern von großer Wichtigkeit.

Der Sterilisierbehälter und seine Teile können aus verschiedenen Metallen bestehen, besonders vorteilhaft ist es aber, wenn dieses Metall Aluminium ist. Dadurch ergeben sich besonders günstige Sterilisationseigenschaften im Hinblick auf die für das Sterilisieren besonders günstigen Werte der Wärmeleitfähigkeit und der Wärmekapazität des Aluminiums. Obwohl Aluminium an sich oberflächlich durch den Sterilisationsvorgang angegriffen würde, schützt eine Polytetrafluorethylenbeschichtung das Aluminium zuverlässig gegen derartige Beschädigungen.

Die nachfolgende Beschreibung einer bevorzugten Ausführungsform der Erfindung dient im Zusammenhang mit der Zeichnung der näheren Erläuterung. Es zeigen:

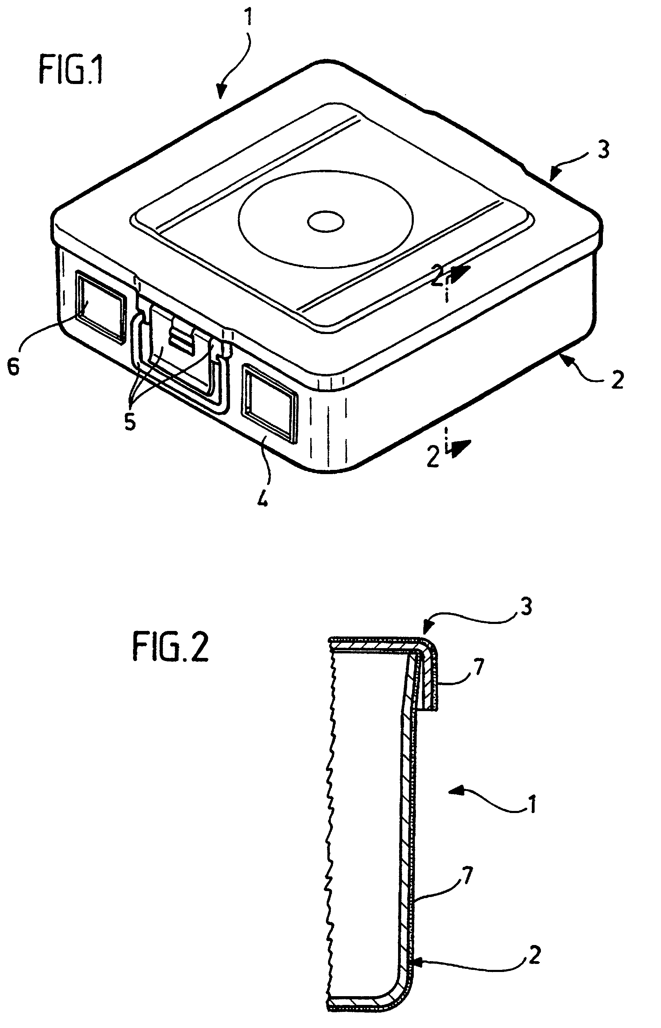

- Figur 1:: eine perspektivische Ansicht eines Sterilisierbehälters und
- Figur 2:: eine Schnittansicht längs Linie 2-2 in Figur 1.

Der in der Zeichnung dargestellte Sterilisierbehälter 1 umfaßt ein wannenförmiges Unterteil 2 und einen dichtend auf dieses aufsetzbaren Deckel 3, die vorzugsweise beide als Aluminiumtiefziehteile ausgebildet sind.

An einer Seitenwand 4 des Unterteils 2 sind Beschlagteile 5 angeordnet, beispielsweise Schließlaschen, Griffbügel und deren Halterungen sowie Einsteckrahmen für Hinweisschilder 6, auf denen Informationen über den Behälterinhalt etc. angeordnet sind.

Sowohl der Deckel 3 als auch das Unterteil 2 sind außenseitig mit einer vollflächigen Beschichtung 7 aus Polytetrafluorethylen versehen, die eine Schichtdicke zwischen 3 um und 50 µm haben kann, vorzugsweise in der Größenordnung von 15 µm bis 20 µm. Dem Polytetrafluorethylenmaterial können Farbstoffe beigemischt sein, so daß der Deckel und/oder das Unterteil dadurch farbig ausgestaltet sind.

In gleicher Weise können einzelne oder alle Beschlagteile 5 und/oder die Hinweisschilder 6 mit Polytetrafluorethylen beschichtet und gegebenenfalls eingefärbt sein.

Die Polytetrafluorethylenbeschichtung hat Antihafteigenschaften und stößt somit Verschmutzungen ab, die Grifffreundlichkeit der Behälter erhöht sich durch diese Beschichtung, und es hat sich überraschenderweise herausgestellt, daß eine solche Beschichtung außerordentlich widerstandsfähig ist, auch dann, wenn beispielsweise ähnlich ausgebildete Behälter aufeinandergesetzt werden. Die Abrieb- und Abkratzeigenschaften sind so, daß die Behälter dabei nicht beschädigt werden. Schließlich wird auch die Lärmbelästigung beim Aneinanderstoßen und Aufeinandersetzen derartiger Behälter durch die Beschichtung herabgesetzt.

## Patentansprüche

1. Sterilisierbehälter mit einem wannenförmigen Unterteil und mit einem dichtend auf dieses aufsetzbaren Deckel, **dadurch gekennzeichnet, daß** das Unterteil (2) und/oder der Deckel (3) allseitig oder außenseitig mit Polytetrafluorethylen beschichtet sind.

2. Sterilisierbehälter nach Anspruch 1, **dadurch gekennzeichnet, daß** Beschlagteile (5) des Sterilisierbehälters (1) mit Polytetrafluorethylen beschichtet sind.

3. Sterilisierbehälter nach einem der voranstehenden Ansprüche, **dadurch gekennzeichnet, daß** Hinweisschilder (6) des Sterilisierbehälters (1) mit Polytetrafluorethylen beschichtet sind.

4. Sterilisierbehälter nach einem der voranstehenden Ansprüche, **dadurch gekennzeichnet, daß** dem Polytetrafluorethylen-Beschichtungsmaterial (7) Farbstoffe beigemischt sind.

5. Sterilisierbehälter nach einem der voranstehenden Ansprüche, **dadurch gekennzeichnet, daß** das Unterteil (2) und/oder der Deckel (3) aus Aluminium bestehen.

6. Sterilisierbehälter nach einem der Ansprüche 2 bis 5, **dadurch gekennzeichnet, daß** die Beschlagteile (5) aus Aluminium bestehen.

7. Sterilisierbehälter nach einem der Ansprüche 3 bis 6, **dadurch gekennzeichnet, daß** die Hinweisschilder (6) aus Aluminium bestehen.

## Claims

1. A sterilisation container comprising a tub-shaped lower part and a cover placeable thereon in a sealed manner, **characterised in that** the lower part (2) and/or the cover (3) are coated on all sides or on the outside with polytetrafluoroethylene.

2. A sterilisation container according to claim 1, **characterised in that** fittings (5) of the sterilisation container (1) are coated with polytetrafluoroethylene.

3. A sterilisation container according to either one of the preceding claims, **characterised in that** information plates (6) of the sterilisation container (1) are coated with polytetrafluoroethylene.

4. A sterilisation container according to any one of the preceding claims, **characterised in that** colorants are added to the polytetrafluoroethylene coating material (7).

5. A sterilisation container according to any one of the preceding claims, **characterised in that** the lower part (2) and/or the cover (3) comprise aluminium.

6. A sterilisation container according to any one of claims 2 to 5, **characterised in that** the fittings (5) comprise aluminium.

7. A sterilisation container according to any one of claims 3 to 6, **characterised in that** the information plates (6) comprise aluminium.

## Revendications

1. Récipient de stérilisation avec une partie inférieure en forme de cuve et avec un couvercle pouvant se poser de manière étanche sur celle-ci, **caractérisé en ce que** la partie inférieure (2) et/ou le couvercle (3) sont revêtus sur tous les côtés ou sur le côté extérieur de polytétrafluoroéthylène.

2. Récipient de stérilisation selon la revendication 1, **caractérisé en ce que** des parties d'armature (5) du récipient de stérilisation (1) sont revêtues de polytétrafluoroéthylène.

3. Récipient de stérilisation selon l'une quelconque des revendications précédentes, **caractérisé en ce que** des plaques indicatrices (6) du récipient de stérilisation (1) sont revêtues de polytétrafluoroéthylène.

4. Récipient de stérilisation selon l'une quelconque des revendications précédentes, **caractérisé en ce que** des colorants sont mélangés avec le matériau de revêtement de polytétrafluoroéthylène (7).

5. Récipient de stérilisation selon l'une quelconque des revendications précédentes, **caractérisé en ce que** la partie inférieure (2) et/ou le couvercle (3) sont constitués d'aluminium.

6. Récipient de stérilisation selon l'une quelconque des revendications 2 à 5, **caractérisé en ce que** les parties d'armature (5) sont constituées d'aluminium.

7. Récipient de stérilisation selon l'une quelconque des revendications 3 à 6, **caractérisé en ce que** les plaques indicatrices (6) sont constituées d'aluminium.
